**Europäisches Patentamt**

**European Patent Office**

**Office européen des brevets**

(11) Veröffentlichungsnummer: **0 483 603 A2**

# EUROPÄISCHE PATENTANMELDUNG

(21) Anmeldenummer: **91117676.6**

(22) Anmeldetag: **16.10.91**

(51) Int. Cl.5: **G01H 11/08**, G10K 11/30

(30) Priorität: **30.10.90 DE 4034533**

(43) Veröffentlichungstag der Anmeldung:
**06.05.92 Patentblatt 92/19**

(84) Benannte Vertragsstaaten:
**DE FR**

(71) Anmelder: **SIEMENS AKTIENGESELLSCHAFT**
**Wittelsbacherplatz 2**
**W-8000 München 2(DE)**

(72) Erfinder: **Granz, Bernd, Dr.**
**Leonhardstrasse 6**
**W-8507 Oberasbach(DE)**
Erfinder: **Köhler, Georg, Dipl.-Ing.**
**Magdalenenstrasse 33**
**W-8618 Geisfeld(DE)**
Erfinder: **Schätzle, Ulrich, Dipl.-Ing.**
**Karl-May-Strasse 14**
**W-8520 Erlangen(DE)**

(54) **Druckimpulsquelle.**

(57) Die Erfindung betrifft eine Druckimpulsquelle mit
einer der Fokussierung der erzeugten Druckimpulse
auf eine Fokuszone dienenden Sammellinse (5), welche wenigstens eine Abbildungszone (IZ) und wenigstens eine Meßzone (MZ2, MZ7) aufweist, wobei in
der Meßzone (MZ2, MZ7) jeweils ein eine piezoelektrische Polymerfolie enthaltender Drucksensor (PS2,
PS7) auf der die Schallaustrittsfläche bildenden
Oberfläche der Sammellinse (5) angebracht ist.

FIG 1

Rank Xerox (UK) Business Services
(−/2.17/2.1)

EP 0 483 603 A2

Die Erfindung betrifft eine Druckimpulsquelle mit einer der Fokussierung der erzeugten Druckimpulse auf eine Fokuszone dienenden Sammellinse und wenigstens einem eine piezoelektrische Polymerfolie enthaltenden Drucksensor, der auf der die Schallaustrittsfläche bildenden Oberfläche der Sammellinse angebracht ist.

Eine derartige Druckimpulsquelle ist in der EP-A-0 229 981 beschrieben. Hier ist eine Vielzahl von Drucksensoren vorgesehen, die dazu dienen, insbesondere auch während des Betriebes der Druckimpulsquelle, die Eigenschaften der Fokuszone, d.h. deren Abmessungen, zu ermitteln. Dabei werden die von den Drucksensoren gelieferten Meßwerte mit Sollwerten verglichen, die einer Fokuszone mit bestimmten Abmessungen entsprechen. Andere wichtige Informationen, z.B. Informationen über den zeitlichen Verlauf und den Spitzenwert des Druckes des aus der Sammellinse austretenden Druckimpulses oder darüber, ob die Druckimpulsquelle relativ zu einem zu beschallenden Objekt derart ausgerichtet ist, daß sich das zu beschallende Objekt bzw. ein bestimmter Bereich des zu beschallenden Objektes in der Fokuszone befindet, können mit der bekannten Druckimpulsquelle nicht oder nicht mit ausreichender Genauigkeit gewonnen werden.

Diese Informationen während des Betriebes der Druckimpulsquelle ständig zur Verfügung zu haben, ist aber insbesondere beim Einsatz der Druckimpulsquelle für medizinische Zwecke, z.B. zur nichtinvasiven Zertrümmerung von Konkrementen, von für die Sicherheit des Patienten elementarer Bedeutung, da einerseits der zeitliche Verlauf des Druckes des aus der Sammellinse austretenden Druckimpulses genaue Rückschlüsse auf den in der Fokuszone zu erwartenden Spitzendruck zuläßt, der zur Vermeidung von unnötigen Gewebeschädigungen nicht höher als unbedingt erforderlich sein sollte. Andererseits soll sich der zu beschallende Bereich stets exakt in der Fokuszone der Druckimpulse befinden, da andernfalls ebenfalls unnötige Gewebeschädigungen zu befürchten sind.

Der Erfindung liegt die Aufgabe zugrunde, eine Druckimpulsquelle der eingangs genannten Art so auszubilden, daß die Voraussetzungen dafür geschaffen sind, während des Betriebes der Druckimpulsquelle eine Kontrolle des Spitzenwertes bzw. des zeitlichen Verlaufes des Druckes der Druckimpulse und/oder der Ausrichtung der Druckimpulsquelle relativ zu dem zu beschallenden Objekt vornehmen zu können.

Nach der Erfindung wird diese Aufgabe gelöst durch eine Druckimpulsquelle mit einer der Fokussierung der erzeugten Druckimpulse auf eine Fokuszone dienenden Sammellinse, welche wenigstens eine Abbildungszone und wenigstens eine Meßzone aufweist, wobei die die Schallaustrittsfläche bildende Oberfläche der Sammellinse in der Meßzone eine von der zur Fokussierung der Druckimpulse erforderlichen Gestalt abweichende Gestalt aufweist und in der Meßzone jeweils ein eine piezoelektrische Polymerfolie enthaltender Drucksensor auf der die Schallaustrittsfläche bildenden Oberfläche der Sammellinse angebracht ist. Die Sammellinse ist also in eine Abbildungszone und eine Meßzone unterteilt, wobei die die Schallaustrittsfläche bildende Oberfläche der Sammellinse nur in der Abbildungszone diejenige Gestalt aufweist, die zur einwandfreien Fokussierung der Druckimpulse auf die Fokuszone erforderlich ist, während sie im Bereich der Meßzone so geformt ist, wie dies der mit dem Drucksensor jeweils durchzuführenden Meßaufgabe optimal entspricht. So ist im Falle einer bevorzugten Variante der Erfindung vorgesehen, daß ein zur Ermittlung des Spitzenwertes bzw. des zeitlichen Verlaufes des Druckes der Druckimpulse vorgesehener Drucksensor auf einer zu der im Bereich der Polymerfolie vorliegenden Schallausbreitungsrichtung wenigstens im wesentlichen rechtwinklig verlaufenden Fläche angebracht ist. Dabei kann eine Auswerteeinrichtung für die von dem Drucksensor gelieferten Signale vorgesehen sein kann, welche den zeitlichen Verlauf der Signale oder wenigstens deren Spitzenwerte ermittelt, wobei im Falle des zeitlichen Verlaufes eine vorzugsweise graphisch und im Falle der Spitzenwerte eine vorzugsweise numerische Anzeige vorgesehen ist. Es kann so der zeitliche Verlauf bzw. der jeweilige Spitzenwert des Druckes eines aus der Sammellinse austretenden Druckimpulses ermittelt werden, wobei infolge der Anordnung des Drucksensors auf einer rechtwinklig zu der Schallausbreitungsrichtung verlaufenden Fläche Meßfehler weitgehend ausgeschlossen sind. Dies ist insbesondere auch hinsichtlich der exakten Wiedergabe des zeitlichen Verlaufes des Druckimpulses wesentlich, da ein unter einem anderen Winkel in bezug auf die Schallausbreitungsrichtung angeordneter Drucksensor infolge des Umstandes, daß er eine endliche Ausdehnung besitzt, Signale liefern würde, die eine gegenüber den tatsächlichen Verhältnissen verlängerte Dauer sowie verringerte Anstiegssteilheit und Spitzenamplitude des Druckimpulses vortäuschen würden. Da eine fortlaufende und zuverlässige Kontrolle der Eigenschaften der in den Patienten eingeleiteten Druckimpulse für die Sicherheit des Patienten unerläßlich ist, sieht eine Ausführungsform der Erfindung vor, daß wenigstens drei Drucksensoren vorgesehen sind, wobei die Auswerteeinrichtung die Spitzenwerte der von den Drucksensoren gelieferten Signale vergleicht und eine Warneinrichtung aktiviert, wenn nicht wenigstens zwei der Spitzenwerte wenigstens im wesentlichen übereinstimmen. Es ist so sichergestellt, daß bei Störungen von mehr als einem Drucksensor das Bedienpersonal darauf hingewiesen wird, daß eine zuverlässige Überwachung der Eigenschaften der Druckimpulse nicht mehr gewährleistet ist.

Gemäß einer weiteren bevorzugten Variante der Erfindung sind wenigstens drei Meßzonen vorgesehen, deren zur Ausrichtung der Druckimpulsquelle und des zu beschallenden Objektes relativ zueinander anhand der reflektierten Anteile der Druckimpulse dienende Drucksensoren auf im Bereich der Polymerfolie wenigstens im wesentlichen sphärisch um die Fokuszone gekrümmten Flächen angebracht sind, welche vorzugsweise wenigstens im wesentlichen den gleichen Krümmungsradius aufweisen. Dabei kann eine Auswerteeinrichtung für die von den Drucksensoren gelieferten Signale vorgesehen sein, welche die zwischen den zu jeweils dem gleichen Druckimpuls gehörigen Ausgangssignalen der Drucksensoren auftretenden Laufzeitdifferenzen auswertet. Beim Auftreffen eines Druckimpulses auf eine Grenzfläche, an der sich die akustische Impedanz sprunghaft ändert, z.B. beim Auftreffen auf die Oberfläche eines Konkrementes, geht von der Grenzfläche eine sich als Kugelwelle ausbreitende Beugungswelle aus. Die zu den einzelnen Drucksensoren gelangenden Anteile der Beugungswelle rufen entsprechende Ausgangssignale der Drucksensoren hervor, deren Laufzeitdifferenzen für den Fall, daß die Beugungswelle von der Fokuszone der Druckimpulse ausgeht, in einer charakteristischen Beziehung zueinander stehen. So sind beispielsweise für den Fall, daß sämtliche Drucksensoren auf sphärisch um die Fokuszone gekrümmten Flächen mit jeweils dem gleichen Krümmungsradius angeordnet sind, die Laufzeiten praktisch identisch, wenn sich das Konkrement in der Fokuszone befindet. Durch Auswertung der Laufzeitdifferenzen kann also festgestellt werden, ob sich ein eine von dem es umgebenden Medium abweichende akustische Impedanz aufweisendes zu beschallendes Objekt, beispielsweise ein Konkrement, in der Fokuszone der Druckimpulsquelle befindet. Dabei ist wesentlich, daß die Drucksensoren auf sphärisch um die Fokuszone gekrümmten Flächen angebracht sind, da sich andernfalls die Impulsdauer der Ausgangssignale der Drucksensoren verlängern und die Anstiegssteilheit und der Spitzenwert der Ausgangssignale verringern würde. Insbesondere die Verringerung der Anstiegssteilheit und des Spitzenwertes hätte jedoch zur Folge, daß bei Laufzeitdifferenzen von beispielsweise 10 $\mu$s, die im menschlichen Gewebe einem Laufwegunterschied von 1,5 cm entsprechen, eine ausreichend exakte Ermittlung der Laufzeitdifferenzen nicht mehr möglich wäre.

Gemäß einer besonders vorteilhaften Variante der Erfindung sind Mittel zum Verstellen der Druckimpulsquelle und eines zu beschallenden Objektes relativ zueinander vorgesehen, wobei die Auswerteeinrichtung die Mittel zum Verstellen derart betätigt, daß die Laufzeitdifferenzen wenigstens im wesentlichen in einer Beziehung zueinander stehen, die einer gewünschten Ausrichtung von Druckimpulsquelle und Objekt relativ zueinander entspricht. Es ist also auf technisch äußerst einfache Weise gewährleistet, daß stets die gewünschte Ausrichtung von Druckimpulsquelle und Objekt relativ zueinander gegeben ist. In diesem Zusammenhang ist es zweckmäßig, wenn vier Drucksensoren vorgesehen sind, die in den Eckpunkten eines Quadrates angeordnet sind, wobei die rechtwinklig zu der Ebene des Quadrates durch dessen Schwerpunkt verlaufende Gerade durch die Fokuszone verläuft. Sind nämlich Druckimpulsquelle und zu beschallendes Objekt in Richtung der Achsen eines rechtwinkligen räumlichen Koordinatensystems relativ zueinander verstellbar und stimmen die Achsen des Koordinatensystems mit den Diagonalen des Quadrates und der durch den Schwerpunkt des Quadrates und die Fokuszone verlaufenden Geraden überein, so kann die korrekte Stellung von Druckimpulsquelle und Objekt relativ zueinander bezüglich zwei der Verstellachsen in einfacher Weise dadurch herbeigeführt werden, indem zunächst durch Verstellen in Richtung der einen Diagonalen des Quadrates die Laufzeitdifferenz zwischen den entsprechenden Drucksensoren zu Null eingestellt wird und dann durch Verstellen in Richtung der anderen Diagonalen die Laufzeitdifferenz zwischen den zugehörigen Drucksensoren ebenfalls zu Null eingestellt wird.

Als Werkstoff für die piezoelektrische Polymerfolie eignet sich insbesondere Polyvinylidenfluorid (PVDF).

Die Erfindung ist in den beigefügten Zeichnungen am Beispiel einer für die nichtinvasive Zertrümmerung von Konkrementen bestimmten Stoßwellenquelle verdeutlicht. Es zeigen:

Fig. 1    einen Längsschnitt durch eine erfindungsgemäße Stoßwellenquelle gemäß der Linie I-I in Fig. 2 in schematischer Darstellung,

Fig. 2    eine Stirnansicht der Sammellinse der erfindungsgemäßen Stoßwellenquelle,

Fig. 3    in schematischer Darstellung einen Drucksensor der erfindungsgemäßen Stoßwellenquelle, und

Fig. 4    in grob schematischer Darstellung ein Blockschaltbild der zu der erfindungsgemäßen Stoßwellenquelle gehörigen Auswerteeinrichtung.

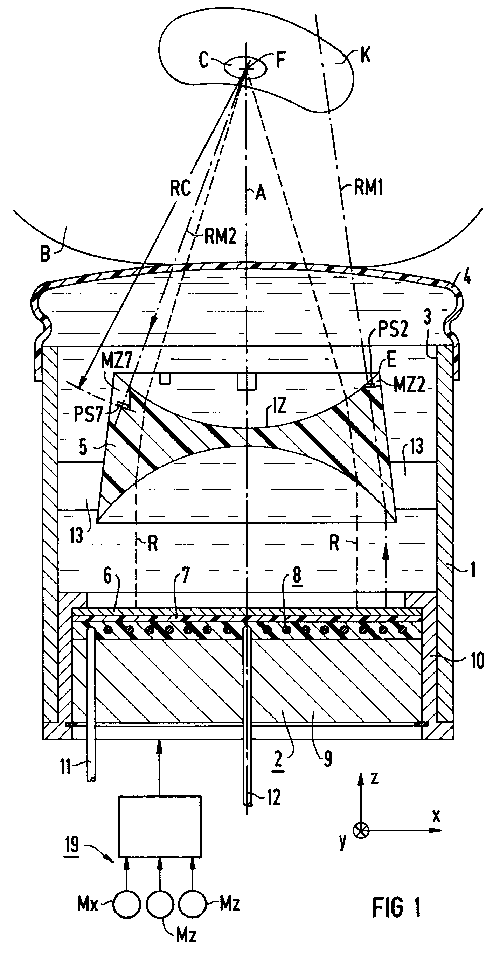

Die Stoßwellenquelle gemäß Fig. 1 weist ein rohrförmiges Gehäuse 1 auf, an dessen einem Ende ein insgesamt mit 2 bezeichneter Stoßwellengenerator angeordnet ist. An dem anderen Ende des Gehäuses I befindet sich eine Austrittsöffnung 3 für von dem Stoßwellengenerator 2 ausgehende Druckimpulse, die mittels eines flexiblen Sackes 4 verschlossen ist. Der von dem Stoßwellengenerator 2, dem Gehäuse 1 und dem flexiblen Sack 4 umschlossener Raum enthält Wasser als akustisches Ausbreitungsmedium für die von dem Stoßwellengenerator 2 ausgehenden Druckimpulse, die sich auf ihrem Ausbreitungsweg infolge der nichtlinearen Kompressionseigenschaften des Ausbreitungsmediums allmählich zu Stoßwellen aufsteilen. Im folgenden wird unabhängig davon, ob ein Druckimpuls sich tatsächlich schon zu einer Stoßwelle aufgesteilt hat, der Einfachheit halber stets der Begriff Stoßwelle verwendet.

Zur Fokussierung der von dem Stoßwellengenerator 2 ausgehenden Stoßwellen ist eine in dem Ausbreitungsmedium angeordnete akustische Sammellinse 5 vorgesehen, welche die Stoßwellen auf eine auf der mit der Mittelachse der Stoßwellenquelle identischen akustischen Achse A der Stoßwellenquelle liegende Fokuszone fokussiert, deren Mittelpunkt mit F bezeichnet ist. Dies ist in Fig. 1 durch zwei strichliert eingetragene "Strahlen" R angedeutet.

Mittels des flexiblen Sackes 4 ist die Stoßwellenquelle an den schematisch angedeuteten Körper B eines Patienten zur akustischen Koppelung anpreßbar. Dabei wird die Stoßwellenquelle so ausgerichtet, daß sich ein im Körper B des Patienten befindliches, zu zertrümmerndes Konkrement C, beispielsweise der Stein einer Niere K, in der Fokuszone befindet. Dies geschieht in an sich bekannter Weise unter Zuhilfenahme einer nicht dargestellten Röntgen-Ortungseinrichtung oder einer vorzugsweise einen Ultraschall-Sektor-Applikator enthaltenden ebenfalls nicht dargestellten Ultraschall-Ortungseinrichtung.

Als Stoßwellengenerator ist ein sogenannter elektromagnetischer Stoßwellengenerator vorgesehen, wie er in der US-PS 4 674 505 näher beschrieben ist. Der Stoßwellengenerator 2 besitzt eine kreisscheibenförmige, ebene Membran 6 aus einem elektrisch leitenden Werkstoff, die mit ihrer einen Seite unmittelbar an das in der Stoßwellenquelle eingeschlossene Wasser grenzt. Der anderen Seite der Membran 6 gegenüberliegend ist unter Zwischenfügung einer Isolierfolie 7 eine insgesamt mit 8 bezeichnete ebene Flächenspule angeordnet, die spiralförmig gewickelt und auf einem Spulenträger 9 aus einem elektrisch isolierenden Werkstoff angebracht ist. Zwischen den spiralförmig verlaufenden Windungen der Flächenspule 8 befindet sich eine elektrisch isolierende Vergußmasse. Die genannten Bauteile des Stoßwellengenerators 2 sind in der Bohrung eines Montageringes 10 axial unverschieblich aufgenommen, der seinerseits in der Bohrung des Gehäuses 1 axial unverschieblich gehaltert ist.

Die Flächenspule 8 weist zwei Anschlüsse 11 und 12 auf, über die sie mit einem in Fig. 1 nicht gezeigten Hochspannungsimpulsgenerator verbunden ist. Dieser beaufschlagt die Flächenspule 8 mit Hochspannungsimpulsen. Wird die Flächenspule 8 mit einem Hochspannungsimpuls beaufschlagt, hat dies zur Folge, daß sie äußerst schnell ein magnetisches Feld aufbaut. Hierdurch wird in die Membran 6 ein Strom induziert, der dem in der Flächenspule 8 fließenden Strom entgegengesetzt ist und demzufolge ein magnetisches Gegenfeld erzeugt, unter dessen Wirkung die Membran 6 schlagartig von der Flächenspule 8 wegbewegt wird. Hierdurch wird eine ebene Stoßwelle in das in der Stoßwellenquelle befindliche Wasser eingeleitet.

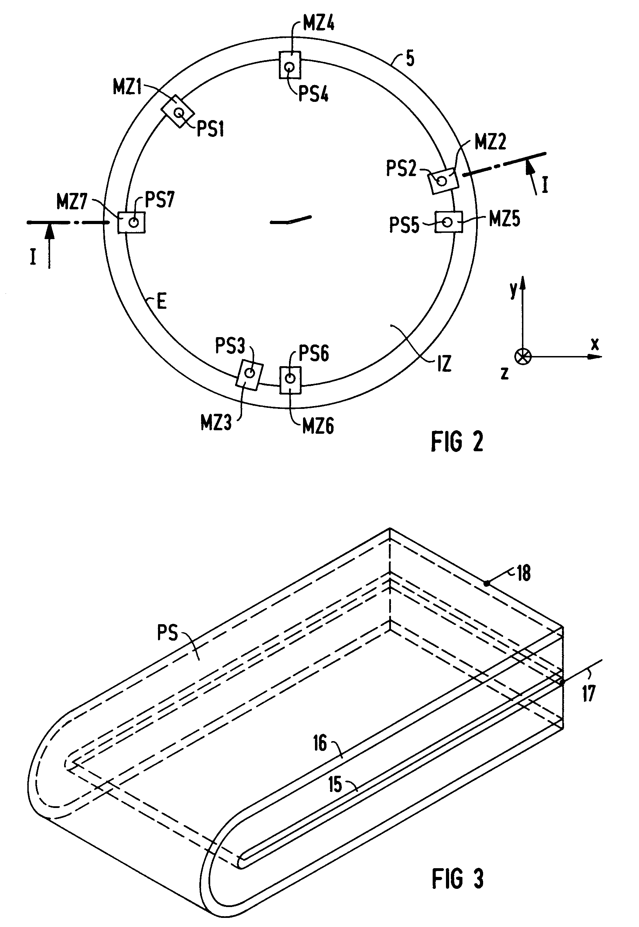

Bei der zur Fokussierung der ebenen Stoßwellen vorgesehenen akustischen Sammellinse 5 handelt es sich um eine zu der akustischen Achse A im wesentlichen rotationssymmetrische bikonkave Linse, die demnach aus einem Material, beispielsweise Polystyrol, gebildet ist, in dem die Schallgeschwindigkeit größer als in dem als akustisches Ausbreitungsmedium vorgesehenen Wasser ist. Die akustische Sammellinse 5 ist mittels einer Anzahl von Tragarmen 13, von denen in Fig. 1 zwei sichtbar sind, in der Bohrung des Gehäuses 1 befestigt. Die Sammellinse 5 ist in der aus Fig. 2 ersichtlichen Weise in eine Abbildungszone IZ und mehrere, nämlich insgesamt sieben auf der von dem Stoßwellengenerator 2 abgewandten Schallaustrittsfläche der Sammellinse 5 vorgesehene Meßzonen MZ1 bis MZ7 unterteilt. Bei der Abbildungszone IZ handelt es sich um denjenigen Bereich der Sammellinse 5, in dem diese, d.h. ihre dem Stoßwellengenerator 2 zugewandte Eintrittsfläche und ihre der Fokuszone zugewandte Austrittsfläche, in einer solchen Weise geformt ist, wie dies für die Fokussierung der Stoßwellen an sich erforderlich ist. In diesem Zusammenhang sei darauf hingewiesen, daß die Gesetze der Strahlenoptik auch in der Akustik gelten. In den Meßzonen MZ1 bis MZ7, in denen jeweils ein in den Fig. 1 und 2 grob schematisch angedeuteter Drucksensor PS1 bis PS7 angeordnet ist, weicht die Gestalt der Sammellinse 5 hinsichtlich der Gestalt ihrer Austrittsfläche, von der zur Fokussierung der Stoßwellen auf die Fokuszone erforderlichen Gestalt ab. Im einzelnen weisen die Meßzonen MZ1 bis MZ3, die identisch ausgebildet und in bezug auf die akustische Achse A um jeweils 120° relativ zueinander versetzt angeordnet sind, eine ebene, den jeweiligen Drucksensor PS1 bis PS3 tragende Fläche auf, die jeweils rechtwinklig zur Schallausbreitungsrichtung des im Bereich der ebenen Fläche aus der Sammellinse 5 austretenden Anteiles der Stoßwelle verläuft. Dies ist im Falle der Fig. 1 anhand der Meßzone MZ2 und des "Strahles" RM1, der einen sich durch die Meßzone MZ2 ausbreitenden Anteil der Stoßwelle veranschaulicht, verdeutlicht. Die Meßzonen MZ4 bis MZ7 weisen jeweils eine kugelförmig gekrümmte Fläche auf, die den jeweiligen Drucksensor PS4 bis PS7 trägt. Die sphärisch gekrümmten Flächen weisen als Krümmungsmittelpunkt jeweils den Mittelpunkt F der Fokuszone auf und besitzen, so wie dies in Fig. 1 für die Meßzone MZ7 angedeutet ist, den Krümmungsradius RC. Dies hat zur Folge, daß, so wie dies am Beispiel des "Strahles" RM2 in Fig. 1 angedeutet ist, eine durch den Mittelpunkt F der Fokuszone verlaufende Gerade rechtwinklig auf der jeweiligen sphärisch gekrümmten Fläche steht. Auch die Meßzonen MZ4 bis MZ7 sind identisch ausgebildet. Sie sind in bezug auf die akustische Achse A um jeweils 90° relativ zueinander versetzt angeordnet. Zwischen der Meßzone MZ1 und der Meßzone MZ7 liegt ein Winkelabstand von 45° vor. Alle Meßzonen MZ1 bis MZ7 sind als in der von dem Stoßwellengenerator 2 abgewandten Randkante E der Sammellinse 5 angebrachte Ausnehmungen ausgeführt.

Der Stoßwellenquelle sind in Fig. 1 grob schematisch angedeutete Verstellmittel 19 mit Elektromotoren Mx, My und Mz zugeordnet. Die Verstellmittel, die in an sich bekannter Weise Getriebe und dergleichen enthalten, dienen dazu, die Stoßwellenquelle in Richtung der Achsen des in Fig. 1 und 2 eingetragenen rechtwinkligen räumlichen Koordiatensystems zu verstellen. Dabei ist der Motor Mx für die Verstellung in Richtung der x-Achse, der Motor My für die Verstellung in Richtung der y-Achse und der Motor Mz für die Verstellung in Richtung der z-Achse des Koordiatensystems zuständig. Die z-Achse entspricht übrigens der akustischen Achse A, während die x-Achse und die y-Achse mittig durch die Meßzonen MZ5 und MZ7 bzw. MZ4 und MZ6 verlaufen. Die Drucksensoren PS4 bis PS7 liegen also in den Eckpunkten eines Quadrates, durch dessen Schwerpunkt die durch die Fokuszone verlaufende akustische Achse A verläuft.

Bei den Drucksensoren, einer davon ist in Fig. 3 schematisch dargestellt, handelt es sich um unter Verwendung von einer piezoelektrisch aktivierten Polymer-Folie gebildete Sensoren, die von der Firma Pennwalt, Großbritannien, unter der Bezeichnung "Kynar-Piezo-Film SDT1-028k" vertrieben werden. Die Piezo-Folie 14 ist beidseitig mit Elektroden 15, 16 versehen, die jeweils aus einer elektrisch leitfähigen, beispielsweise metallischen Schicht bestehen. Die mit den Elektroden 15, 16 versehene Piezo-Folie 14 ist in einer solchen Weise U-förmig gefaltet, daß die einander zugewandten Seiten der Elektrode 15 aneinander anliegen. Beide Elektroden 15 und 16 sind mit Anschlußleitungen 17, 18 versehen. die zu einer noch zu beschreibenden Auswerteeinrichtung führen. Die Wirkungsweise derartiger an sich bekannter Drucksensoren beruht darauf, daß durch die Einwirkung von Stoßwellen ein Wechselladungssignal erzeugt wird, das von der Piezo-Folie 14 mittels der Elektroden 15, 16 abgenommen wird. Da die Piezo-Folie 14 hinsichtlich ihrer akustischen Impedanz im Falle der Verwendung von Polystyrol als Material für die Sammellinse 5 und von Wasser als akustisches Ausbreitungsmedium an deren akustische Impedanzen weitgehend angepaßt ist und die Elektroden 15, 16 sehr dünn sind, treten an den Grenzflächen zwischen der akustischen Sammellinse 5 und den Drucksensoren PS1 bis PS7 sowie an den Grenzflächen zwischen diesen und dem Wasser nur sehr schwache Reflexionen auf, so daß diese in den Ausgangssignalen der Drucksensoren PS1 bis PS7 praktisch nicht repräsentiert sind. Die unter Verwendung der polymeren Piezo-Folie gebildeten Drucksensoren PS1 bis PS7 bieten außerdem den Vorteil, daß sie wegen der hohen Verformbarkeit solcher Polymerfolien leicht auf den im Bereich der Meßzonen MZ4 bis MZ7 vorgesehenen sphärisch gekrümmten Flächen, beispielsweise durch Kleben, befestigt werden können. Außerdem hat sich gezeigt, daß derartige Drucksensoren äußerst dauerhaft sind und Ausgangssignale mit Amplituden in der Größenordnung einige Volt abgeben, so daß ein sehr gutes Signal/Rausch-Verhältnis erzielt wird.

Die den Meßzonen MZ1 bis MZ3 zugeordneten Drucksensoren PS1 bis PS3 dienen dazu, den zeitlichen Verlauf und den Spitzenwert des Druckes der im Bereich der Meßzonen MZ1 bis MZ3 aus der Sammellinse 5 austretenden Anteile der jeweiligen Stoßwelle zu messen. Es ist so möglich, die Funktion der Stoßwellenquelle anhand von Kenngrößen der erzeugten Stoßwellen kontinuierlich zu überwachen, so daß eventuelle Funktionsstörungen der Stoßwellenquelle bereits in einem sehr frühen Stadium erkannt werden können. Außerdem ist es möglich, aus den gemessenen Kenngrößen der Stoßwellen Rückschlüsse auf die Verhältnisse in der Fokuszone zu ziehen, was im Interesse der Sicherheit des Patienten von besonderer Bedeutung ist. Dabei ist es zweckmäßig, eine Eichung der Drucksensoren PS1 bis PS3 vorzunehmen, so daß der Druck nicht nur qualitativ, sondern quantitativ gemessen werden kann.

Die den Meßzonen MZ4 bis MZ7 zugeordneten Drucksensoren PS4 bis PS7 dienen dazu, Laufzeitdifferenzen zwischen den zu den Drucksensoren PS4 bis PS7 gelangenden Anteilen der nach dem Auftreffen einer Stoßwelle auf das zu zertrümmernde Konkrement C von diesem ausgehenden kugelwellenförmigen Beugungswelle zu ermitteln. Anhand dieser Laufzeitdifferenzen, die bei der beschriebenen Anordnung und Ausbildung der Meßzonen MZ4 bis MZ7 dann verschwinden, wenn das zu zertrümmernde Konkrement C auf der akustischen Achse A liegt, besteht die Möglichkeit, die Ausrichtung der Stoßwellenquelle relativ zu dem zu zertrümmernden Konkrement C zu kontrollieren und erforderlichenfalls zu korrigieren.

Meßfehler, die sich in einer Verlängerung der Impulsdauer und einer Verringerung der Anstiegssteilheit und der Spitzenwerte der Angangssignale der Drucksensoren PS1 bis PS3 bzw. PS4 bis PS7 gegenüber dem tatsächlichen Verlauf des Druckes der gemessenen Anteile der Stoßwellen bzw. der Beugungswellen äußern würden, sind infolge der beschriebenen Anordnung der Drucksensoren PS1 bis PS3 bzw. PS4 bis PS7 auf rechtwinklig zur jeweiligen Schallausbreitungsrichtung verlaufenden bzw. sphärisch um das Zentrum der Fokuszone gekrümmten Flächen vermieden.

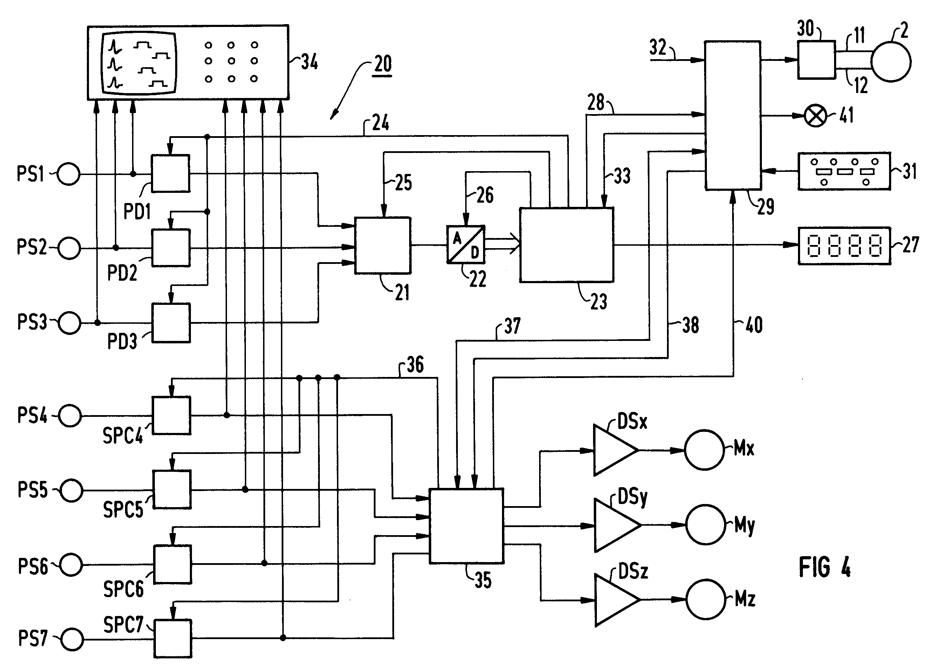

Mit der Stoßwellenquelle 2, den Drucksensoren PS1 bis PS7 und den Motoren Mx, My und Mz der Verstellmittel 19 zusammenwirkende, insgesamt mit 20 bezeichnete Auswerteeinrichtung für die Ausgangssignale der Drucksensoren PS1 bis PS7 ist in Fig. 4 dargestellt. Demnach sind die Drucksensoren PS1 bis PS3 jeweils an einen Spitzenwertdetektor und -speicher PD1 bis PD3 angeschlossen. Deren Ausgangssignale sind den Eingängen eines 3 zu 1-Analogmultiplexers 21 zugeführt, dessen Ausgang mit dem Eingang eines Analog/Digital-Wandlers 22 verbunden ist. Die digitalen Ausgangsdaten des Analog/Digital-Wandlers 22 sind einer Rechen- und Steuerungseinheit 23 zugeführt. Diese steuert über Steuerleitungen 24, 25 und 26 die Spitzenwertdetektoren und -speicher PD1 bis PD3, den Analogmultiplexer 21 und den Analog/Digital-Wandler 22. Dabei erfolgt die Steuerung in der Weise, daß der in den Spitzenwertdetektoren und speichern PD1 bis PD3 zuletzt gespeicherte Spitzenwert des Ausgangssignales des jeweiligen Drucksensors PS1 bis PS3 unmittelbar vor Erzeugung einer Stoßwelle mittels des Stoßwellengenerators 2 gelöscht wird. Eine kurze Zeitspanne, die wenigstens gleich der Laufzeit der Stoßwelle von dem Stoßwellengenerator 2 durch die Sammellinse 5 hindurch ist, nach erfolgter Abgabe der Stoßwelle führt der Analogmultiplexer 21 nacheinander die mittels der Spitzenwertdetektoren und -speicher PD1 bis PD3 detektierten und gespeicherten Spitzenwerte der Ausgangssignale der Drucksensoren PS1 bis PS3 dem Analog/Digital-Wandler 22 zu. Die entsprechenden digitalen Daten gelangen aufeinanderfolgend in die Steuer- und Recheneinheit 23, wo sie zwischengespeichert werden. Die Steuer- und Recheneinheit 23 vergleicht die ermittelten Spitzenwerte miteinander und stellt fest, ob wenigstens zwei der ermittelten Spitzenwerte im wesentlichen übereinstimmen. Dabei soll unter wenigstens im wesentlichen übereinstimmenden Spitzenwerten verstanden werden, daß der niedrigere Spitzenwert um beispielsweise maximal 10% von dem höheren Spitzenwert abweicht. Stimmen wenigstens zwei Spitzenwerte im wesentlichen überein, ermittelt die Steuer- und Recheneinheit 23 den Mittelwert der im wesentlichen übereinstimmenden Spitzenwerte, multipliziert diesen mit einem in der Steuer- und Recheneinheit 23 gespeicherten Eichfaktor und zeigt den dadurch erhaltenen Mittelwert der mit den Drucksensoren PS1 bis PS3 gemessenen, im wesentlichen übereinstimmenden Spitzendrücke auf einer an die Steuer- und Recheneinheit angeschlossenen Anzeigeeinrichtung 27 an. Im Falle der Fig. 4 ist eine digitale Anzeigeeinrichtung dargestellt. Es können jedoch auch analoge Anzeigeeinrichtungen vorgesehen sein. Stellt die Steuer- und Recheneinheit 23 fest, daß nicht wenigstens zwei der Spitzenwerte wenigstens im wesentlichen übereinstimmen, gibt sie ein entsprechendes Signal über eine Leitung 28 an eine Steuereinrichtung 29, die daraufhin eine Warneinrichtung 41 aktiviert. Im Falle der Fig. 4 ist als Warneinrichtung 41 ein Lichtsignal dargestellt.

An die Steuereinrichtung 29 sind außerdem der zum Betrieb der Stoßwellenquelle erforderliche Hochspannungsimpulsgenerator 30, der mit den Anschlüssen 11 und 12 der Stoßwellenquelle 12 verbunden ist, über eine Steuerleitung angeschlossen. Über diese aktiviert die Steuereinrichtung 29 den Hochspannungsimpulsgenerator 30 zur Abgabe von Hochspannungsimpulsen, die ihrerseits zur Erzeugung von Stoßwellen führen. Die Aktivierung des Hochspannungsimpulsgenerators 30 kann wahlweise periodisch mittels der Steuereinrichtung 29, manuell mittels einer an die Steuereinrichtung 29 angeschlossenen Tastatur 31 oder in an sich bekannter Weise anhand eines der Steuereinrichtung 29 über einen Anschluß 32 zuführbaren, eine periodische Körperfunktion des Patienten repräsentierenden Signales erfolgen.

Die beschriebenen Vorgänge der Ermittlung und Speicherung der Spitzenwerte der Ausgangssignale der Drucksensoren PS1 bis PS3, deren Analog/Digital-Wandlung und die Verarbeitung und gegebenenfalls Anzeige der erhaltenen digitalen Daten mittels der Steuer- und Recheneinheit 23 wiederholen sich jeweils im Anschluß an die Erzeugung einer Stoßwelle. Die hierzu erforderlichen Taktsignale erhält die Steuer- und Recheneinheit 23 von der Steuereinrichtung 29 über eine Leitung 33.

Die Ausgangssignale der Drucksensoren PS1 bis PS3 sind einem Vielkanal-Oszilloskop 34 zugeführt, das diese in der linken Hälfte seines Schirmes vertikal übereinander darstellt, so daß auch eine Kontrolle des zeitlichen Verlaufes des Druckes der Stoßwellen möglich ist.

Die Ausgangssignale der Drucksensoren PS4 bis PS7 sind jeweils einer Signalaufbereitungsschaltung SPC4 bis SPC7 zugeführt. Diese werden von einer Steuer- und Zeitmeßeinheit 35 über eine Steuerleitung 36 derart gesteuert, daß ihre Eingänge bei Erzeugung einer Stoßwelle für eine Zeit gesperrt sind, die mindestens gleich der Laufzeit der Stoßwelle von dem Stoßwellengenerator 2 durch die Sammellinse 5 hindurch entspricht und die nicht wesentlich länger als die Laufzeit der Stoßwelle von dem Stoßwellengenerator 2 zu dem zu zertrümmernden Konkrement C ist. Die hierzu erforderlichen Taktsignale erhält die Steuer- und Zeitmeßeinheit 35 über eine Leitung 37 von der Steuereinrichtung 29. Es können also nur diejenigen Anteile der Ausgangssignale der Drucksensoren PS4 bis PS7 in die Signalaufbereitungsschaltungen SPC4 bis SPC7 gelangen, die die von dem zu zertrümmernden Konkrement C nach Beaufschlagung mit einer Stoßwelle ausgehende Beugungswelle repräsentieren. Diese Signalanteile werden in den identischen Signalaufbereitungsschaltungen SPC4 bis SPC7 beispielsweise mittels eines Schmitt-Triggers mit nachgeschaltetem Monoflop in Rechteckimpulse einer definierten Dauer umgewandelt, wobei die Dauer der Rechteckimpulse größer als die Gesamtdauer der Beugungswelle ist. Hierdurch wird erreicht, daß jede Beugungswelle die in den Signalaufbereitungsschaltungen SPC4 bis SPC7 enthaltenen Monoflops nur ein einziges Mal triggern kann. Die genannten Rechteckimpulse sind der Steuer- und Zeitmeßeinheit 35 zugeführt. Diese mißt zum einen die Zeitdauer, um die die Anstiegsflanken der von den Signalaufbereitungsschaltungen SPC5 und SPC7 kommenden Rechteckimipulse gegeneinander versetzt sind. Diese Zeitdauer entspricht der Laufzeitdifferenz zwischen denjenigen Anteilen der Beugungswelle, die zu den auf der x-Achse liegenden Drucksensoren PS5 und PS7 gelangen. Zum anderen mißt die Steuer- und Zeitmeßeinheit 35 die Zeitdauer, um die die Anstiegsflanken der von den Signalaufbereitungsschaltungen SPC4 und SPC6 stammenden Rechteckimpulse zueinander verstetzt sind. Diese Zeitdauer entspricht dann der Laufzeitdifferenz zwischen den auf der y-Achse liegenden Drucksensoren PS4 und PS6 gelangenden Anteilen der Beugungswelle. Schließlich mißt die Steuer-und Zeitmeßeinheit 35 diejenige Zeitdauer, die zwischen der Aktivierung des Stoßwellengenerators 2 zur Erzeugung einer Stoßwelle, ein entsprechendes Signal ist der Steuer- und Zeitmeßeinheit 35 von der Steuereinrichtung 29 zugeführt, und dem Eintreffen der Anstiegsflanke des aus dem Ausgangssignal eines der Drucksensoren PS4 bis PS7, beispielsweise des Drucksensors PS4, gebildeten Rechteckimpulses verstreicht. Zwischen der so gemessenen Zeitdauer und einem in der Steuer- und Zeitmeßeinheit 35 gespeicherten Wert, der der Summe der Laufzeiten der Stoßwelle von dem Stoßwellengenerator zu dem zu zertrümmernden Konkrement C und der Laufzeit der Beugungswelle von dem zu zertrümmernden Konkrement C zu dem Drucksensor PS4 bei exakt in der Fokuszone befindlichem zu zertrümmernden Konkrement C entspricht, ermittelt die Steuer- und Zeitmeßeinheit die Zeitdifferenz. Anhand der wie beschrieben ermittelten Daten betätigt die Steuer- und Recheneinheit 35 über Treiberstufen DSx, DSy, DSz die Motore Mx, My und Mz der Verstellmittel 19 in einer solchen Weise, daß die Laufzeitdifferenzen der Beugungswelle zu den Drucksensoren PS4 und PS6 bzw. PS5 und PS7 ebenso wie die Zeitdifferenz zwischen dem gespeicherten Wert und der Zeitdauer zwischen der Abgabe einer Stoßwelle und dem Eintreffen der Beugungswelle an dem Drucksensor PS4 verschwinden. Dies entspricht einer exakten Ausrichtung der Stoßwellenquelle auf das zu zertrümmernde Konkrement C. Dabei geht die Steuer- und Zeitmeßeinrichtung derart vor, daß sie zunächst durch schrittweises Ansteuern der Motore Mx und My mit einer Schrittweite von beispielsweise 1 mm im Anschluß an jede Stoßwelle die Stoßwellenquelle in der x-y-Ebene derart ausrichtet, daß die genannten Laufzeitdifferenzen verschwinden. Dabei ergibt sich die jeweilige Verstellrichtung aus dem Vorzeichen der Laufzeitdifferenz, welches die Steuer- und Recheneinheit 35 daran erkennt, ob die Anstiegsflanke des zu dem Drucksensor PS4 oder des zu dem Drucksensor PS6 bzw. die Anstiegsflanke des zu dem Drucksensor PS5 bzw. des zu dem Drucksensor PS7 gehörigen Rechteckimpulses zuerst eintrifft. Im Anschluß hieran erfolgt die Verstellung der Stoßwellenquelle in z-Richtung, wobei sich auch hier die Verstellrichtung aus dem Vorzeichen der wie beschrieben ermittelten Zeitdifferenz ergibt.

Um eine optische Kontrolle über die Ausrichtung der Stoßwellenquelle relativ zu dem zu zertrümmernden Konkrement C zu ermöglichen, sind die Ausgangssignale der Signalaufbereitungsschaltungen SPC4 ebenfalls dem Vielkanal-Oszilloskop 34 zugeführt, wo sie in der rechten Hälfte des Bildschirmes in korrekter Phasenlage vertikal übereinander dargestellt werden.

Stellt die Steuer- und Recheneinheit 35 fest, daß einer der Drucksensoren PS4 bis PS7 ausgefallen ist, was sie an dem Ausbleiben des entsprechenden Rechteckimpulses erkennt, gibt sie über eine Leitung 40 ein entsprechendes Signal an die Steuereinrichtung 29, die daraufhin die Warneinrichtung 41 betätigt.

Es versteht sich, daß die beschriebene Ausrichtung der Stoßwellenquelle relativ zu dem zu zertrümmernden Konkrement C mit Hilfe der Ausgangssignale der Drucksensoren PS4 bis PS7 nur zur Korrektur geringfügiger, während der Behandlung auftretender Abweichungen dienen kann und daß die eigentliche Ortung des zu zertrümmernden Konkrementes zuvor mit Hilfe einer Röntgen- oder Ultraschall-Ortungseinrichtung erfolgen muß. Es ist jedoch auch eine Ortung des zu zertrümmernden Konkrementes C vor Beginn der Behandlung anhand der Ausgangssignale der Drucksensoren PS4 bis PS7 möglich. In diesem Falle muß jedoch die Stoßwellenquelle derart betrieben werden, daß sie Stoßwellen geringer Intensität abgibt, so daß Schädigungen des Patienten ausgeschlossen sind. Erst nach erfolgter Ortung des zu zertrümmernden Konkrementes C werden dann Stoßwellen einer solchen Intensität erzeugt, die zur Zertrümmerung des Konkrementes C ausreicht. Stoßwellen verringerter Intensität können beispielsweise dadurch erzeugt werden, daß der Hochspannungsimpulsgenerator 30 den Stoßwellengenerator 2 mit Hochspannungsimpulsen verringerter Amplitude beaufschlagt.

Im Falle des beschriebenen Ausführungsbeispieles weist die Sammellinse 5 nur eine einzige Abbildungszone IZ auf. Es besteht aber die Möglichkeit, auch mehrere Abbildungszonen vorzusehen. Als wesentlicher Vorteil der erfindungsgemäßen Stoßwellen- bzw. einem Drucksensor PS1 bis PS7 versehene Meßzonen MZ1 bis MZ3 bzw. MZ4 bis MZ7 unterschiedlicher geometrischer Ausbildung in jeweils optimaler Weise die Überwachung der erzeugten Druckimpulse bzw. Stoßwellen hinsichtlich des Spitzenwertes bzw. des zeitlichen Verlaufes ihres Druckes bzw. die Kontrolle der Ausrichtung der Quelle relativ zu einem zu beschallenden Objekt während des Betriebes der Druckimpuls- bzw. Stoßwellenquelle möglich ist.

Im Falle des beschriebenen Ausführungsbeispieles ist als Druckimpulsquelle eine elektromagnetische Stoßwellenquelle vorgesehen. Es können jedoch auch andere, beispielsweise piezoelektrische Druckimpulsquellen verwendet werden.

**Patentansprüche**

1. Druckimpulsquelle mit einer der Fokussierung der erzeugten Druckimpulse auf eine Fokuszone dienenden Sammellinse (5), welche wenigstens eine Abbildungszone (IZ) und wenigstens eine Meßzone (MZ1 bis MZ7) aufweist, wobei die die Schallaustrittsfläche bildende Oberfläche der Sammellinse (5) in der Meßzone eine von der zur Fokussierung der Druckimpulse erforderlichen Gestalt abweichende Gestalt aufweist und in der Meßzone (MZ1 bis MZ7) jeweils ein eine piezoelektrische Polymerfolie (14) enthaltender Drucksensor (PS1 bis PS7) auf der die Schallaustrittsfläche bildenden Oberfläche der Sammellinse (5) angebracht ist.

2. Druckimpulsquelle nach Anspruch 1, **dadurch gekennzeichnet**, daß der Drucksensor (PS1 bis PS3) auf einer zu der im Bereich der Polymerfolie (14) vorliegenden Schallausbreitungsrichtung der Druckimpulse wenigstens im wesentlichen rechtwinklig verlaufenden Fläche angebracht ist.

3. Druckimpulsquelle nach Anspruch 2, **dadurch gekennzeichnet**, daß eine Auswerteeinrichtung (20) für die von dem Drucksensor (PS1 bis PS3) gelieferten Signale vorhanden ist, welche den zeitlichen Verlauf der Signale oder wenigstens deren Spitzenwerte ermittelt.

4. Druckimpulsquelle nach Anspruch 3, **dadurch gekennzeichnet**, daß wenigstens drei Drucksensoren (PS1 bis PS3) vorgesehen sind, und daß die Auswerteeinrichtung (20) die Spitzenwerte der von den Drucksensoren (PS1 bis PS3) gelieferten Signale vergleicht und eine Warneinrichtung (41) aktiviert, wenn nicht wenigstens zwei Spitzenwerte wenigstens im wesentlichen übereinstimmen.

5. Druckimpulsquelle nach einem der Ansprüche 1 bis 4, **dadurch gekennzeichnet**, daß wenigstens drei Meßzonen (MZ4 bis MZ7) vorgesehen sind, deren Drucksensoren (PS4 bis PS7) auf im Bereich der jeweiligen Polymerfolie (14) wenigstens im wesentlichen sphärisch um die Fokuszone gekrümmten Flächen angebracht sind.

6. Druckimpulsquelle nach Anspruch 5, **dadurch gekennzeichnet**, daß die Flächen wenigstens im wesentlichen den gleichen Krümmungsradius (RC) aufweisen.

7. Druckimpulsquelle nach Anspruch 5 oder 6, **dadurch gekennzeichnet**, daß eine Auswerteeinrichtung (20) für die von den Drucksensoren (PS4 bis PS7) gelieferten Signale vorgesehen ist, welche die zwischen den zu jeweils dem gleichen Druckimpuls gehörigen Ausgangssignalen der Drucksensoren (PS4 bis PS7) auftretenden Laufzeitdifferenzen auswertet.

8. Druckimpulsquelle nach Anspruch 7, dadurch ge**kennzeichnet**, daß Mittel (19) zum Verstellen der Druckimpulsquelle und eines zu beschallenden Objektes (B, C) relativ zueinander vorgesehen sind und daß die Auswerteeinrichtung (20) die Mittel (19) zum Verstellen derart betätigt, daß die Laufzeitdifferenzen wenigstens im wesentlichen in einem Verhältnis zueinanderstehen, daß einer gewünschten Ausrichtung von Druckimpulsquelle und Objekt (B, C) relativ zueinander entspricht.

9. Druckimpulsquelle nach einem der Ansprüche 5 bis 8, **dadurch gekennzeichnet**, daß vier Drucksensoren (PS4 bis PS7) vorgesehen sind, die in den Eckpunkten eines Quadrates angeordnet sind, wobei die rechtwinklig zu der Ebene des Quadrates durch dessen Schwerpunkt verlaufende Gerade durch die Fokuszone verläuft.

10. Druckimpulsquelle nach einem der Ansprüche 1 bis 9, **dadurch gekennzeichnet**, daß als piezoelektrische Polymerfolie (14) eine Polyvinylidenfluorid (PVDF)-Folie vorgesehen ist.

FIG 1

FIG 2

FIG 3

11

FIG 4

EP 0 483 603 A2